# EUROPÄISCHE PATENTANMELDUNG

(11) **EP 0 992 498 A1**
(43) Veröffentlichungstag der Anmeldung: **12.04.2000**
(21) Anmeldenummer: 00100640.2
(22) Anmeldetag: 28.10.1992
(51) Int. Cl.: C07D 239/26, C09K 19/34, C07D 239/34, C07D 213/30, G02F 1/13

(54) **Aromatische Fettsäureester**

(30) Priorität: 08.11.1991 CH 327391; 18.06.1992 CH 192792
(62) Teilanmeldung aus: 92118359.6
(71) Anmelder: Rolic AG, 6301 Zug (CH)
(72) Erfinder: Kelly, Stephen, 4313 Möhlin (DE)
(74) Vertreter: Eder, Carl E.

(57) **Zusammenfassung**

In der vorliegenden Erfindung werden Verbindungen der allgemeinen Formel 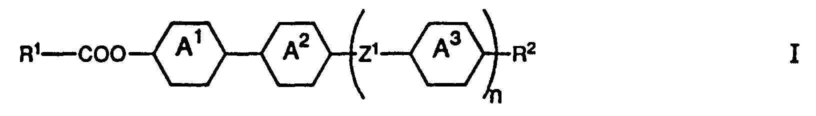 beschrieben, für welche
- R^{l}: unsubstituiertes oder einfach oder mehrfach mit Halogen substituiertes Alkyl oder Alkenyl mit l bis 12, bzw. 2 bis 12 Kohlenstoffatomen, in welchen eine Methylengruppe durch -O- ersetzt sein kann, bedeutet;
- R²: unsubstituiertes oder einfach oder mehrfach mit Halogen substituiertes Alkyl oder Alkenyl mit l bis 12, bzw. 2 bis 12 Kohlenstoffatomen, in welchen eine oder mehrere Methylengruppen durch -O- und/oder -COO- oder -OOC- ersetzt sein können, bedeutet;
- Z¹: eine einfache Kovalenz-Bindung oder -CH₂CH₂-bedeutet;
- A¹, A²: unabhängig voneinander unsubstituiertes oder einfach oder mehrfach mit Halogen substituiertes 1,4-Phenylen darstellen;
- A³: Pyridin-2,5-diyl oder Pyrimidin-2,5-diyl darstellt; und
- n: 1 ist.
Ebenfalls beschrieben sind die Herstellung und Verwendung solcher Verbindungen in elektro-optischen Vorrichtungen, sowie Mischungen, die diese Verbindungen enthalten.

## Beschreibung

Die vorliegende Erfindung betrifft aromatische Ester, flüssigkristalline Gemische, die solche Verbindungen enthalten sowie die Verwendung solcher Ester für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), SSF-Zellen (surface stabilized ferroelectric), DHF-Zellen (deformed helix ferroelectric) oder SBF-Zellen (short-pitch bistable ferroelectric).

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine breite smektische Mesophase.

Da Flüssigkristalle in der Regel als Mischungen mehrerer Komponenten zur Anwendung gelangen, ist es wichtig, dass die Komponenten untereinander gut mischbar sind.

Die vorliegende Erfindung stellt nun Verbindungen, die in hervorragender Weise für solche Flüssigkristall-Mischungen geeignet sind, zur Verfügung. Gegenstand der vorliegenden Erfindung sind Verbindungen der allgemeinen Formel 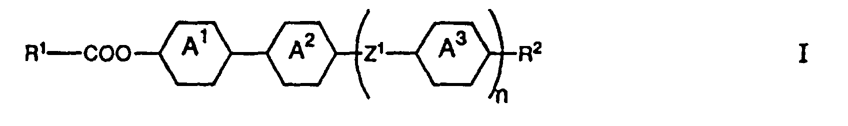 worin
- R^{l}: unsubstituiertes oder einfach oder mehrfach mit Halogen substituiertes Alkyl oder Alkenyl mit l bis 12, bzw. 2 bis 12 Kohlenstoffatomen, in welchen eine Methylengruppe durch -O- ersetzt sein kann, bedeutet;
- R²: unsubstituiertes oder einfach oder mehrfach mit Halogen substituiertes Alkyl oder Alkenyl mit l bis 12, bzw. 2 bis 12 Kohlenstoffatomen, in welchen eine oder mehrere Methylengruppen durch -O- und/oder -COO- oder -OOC- ersetzt sein können, bedeutet;
- Z¹: eine einfache Kovalenz-Bindung oder -CH₂CH₂- bedeutet;
- A¹, A²: unabhängig voneinander unsubstituiertes oder einfach oder mehrfach mit Halogen substituiertes 1,4-Phenylen, oder Pyridin-2,5-diyl oder Pyrimidin-2,5-diyl darstellen;
- A³: unsubstituiertes oder einfach oder mehrfach mit Halogen substituiertes 1,4-Phenylen, oder Pyridin-2,5-diyl, Pyrimidin-2,5-diyl oder trans-l,4-Cyclohexylen darstellt;
- n: 0 oder l ist;
mit der Massgabe, dass mindestens einer der Ringe A¹, A² oder A³ entweder Pyridin-2,5-diyl oder Pyrimidin-2,5-diyl bedeutet; und mit der weiteren Massgabe, dass falls n = 0 ist, R¹ 1E-Alkenyl und R² Alkyl, Alkoxy oder Alkenyloxy darstellen.

Es wurde überraschenderweise gefunden, dass die erfindungsgemässen Verbindungen für ferroelektrische Anwendungen ausgesprochen günstige Mesophasen aufweisen.

Durch Beimischen der erfindungsgemässen Verbindungen der allgemeinen Formel I wird in Flüssigkristall-Mischungen oft eine bedeutende Erhöhung des S_{C}-S_{A}, S_{C}-N, S_{A}-I oder N-I Phasenüberganges bewirkt und der Schmelzpunkt signifikant abgesenkt, was zu einer breiten smektischen Mesophase führt. Die erfindungsgemässen Verbindungen weisen breite smektische Mesophasen mit erstaunlich niedriger Viskosität auf. Dadurch können durch Zugabe solcher Verbindungen die Schaltzeiten von Grundmischungen erheblich gesenkt werden. Die erfindungsgemässen zweikernigen Verbindungen erweisen sich trotz ihrer nematischen Phase als ausgezeichnete Komponenten für S_{C}-Mischungen. Sie verschmälern die S_{A}-Phase und induzieren eine nematische Phase, ohne den S_{C}-Phasen-Über-gang zu senken. Auf diese Weise ergeben sich Phasen, die sich optimal ausrichten lassen, und die sich so hervorragend für SSF Anwendungen eignen.

Der Ausdruck "unsubstituiertes oder einfach oder mehrfach mit Halogen substituiertes Alkyl oder Alkenyl mit l bis 12, bzw. 2 bis 12 Kohlenstoffatomen, in welchen eine Methylengruppe durch -O- ersetzt sein kann" bedeutet im Rahmen der vorliegenden Erfindung Gruppen wie Alkyl, Alkoxy, Alkenyl, Alkenyloxy, Alkoxyalkyl, Alkenyloxyalkyl, Alkoxyalkenyl mit l bis 12, bzw. 2 bis 12 Kohlenstoffatomen. Diese Gruppen können unverzweigt oder verzweigt sein; bevorzugt werden die unverzweigten. Diese Gruppen können einfach oder mehrfach mit Halogen, insbesondere mit Fluor, substituiert sein. Solche Gruppen sind beispielsweise Methyl, Aethyl, Propyl, Butyl, Pentyl, Hexyl, Isopropyl, 2-Methyl-butyl, 2-Methyl-pentyl, 3-Methyl-butyl, 3-Methyl-pentyl, 4-Methyl-pentyl, Methoxy, Aethoxy, Propyloxy, Butyloxy, Pentyloxy, Hexyloxy, Methoxy-propyl, Methoxybutyl, Methoxy-pentyl, Aethoxypropyl, Aethoxybutyl, Propyloxypropyl, 1E-Propenyl, 1E-Butenyl, 1E-Pentenyl, 1E-Hexenyl, 1E-Heptenyl, 1E-Octenyl, 1E-Nonenyl, 1E-Decenyl, 1E-Undecenyl und dergleichen.

Der Ausdruck "unsubstituiertes oder einfach oder mehrfach mit Halogen substituiertes Alkyl oder Alkenyl mit l bis 12, bzw. 2 bis 12 Kohlenstoffatomen, in welchen eine oder mehrere Methylengruppen durch -O- und/oder -COO- oder -OOC- ersetzt sein können" bedeutet im Rahmen der vorliegenden Erfindung u.a. Gruppen wie vorhergehend definiert. Zusätzlich umfasst dieser Ausdruck Alkenylgruppen wie 2Z-Butenyl, 2Z-Pentenyl, 2Z-Hexenyl, 3-Butenyl, 3E-Pentenyl, 3E-Hexenyl, 4-Pentenyl, 4Z-Hexenyl, 5-Hexenyl, Allyloxy, 2E-Butenyloxy, 3-Butenyloxy, 2E-Pentenyloxy, 3Z-Pentenyloxy, 4-Pentenyloxy, 2E-Hexenyloxy, 3Z-Hexenyloxy, 4E-Hexenyloxy, 2E-Heptenyloxy, 2E-Octenyloxy, 2E-Nonenyloxy, 2E-Decenyloxy, 2E-Undecenyloxy, 2E-Dodecenyloxy. Allyloxypropyl, 3-Butenyloxypropyl, und auch verzweigte oder vorzugsweise unverzweigte Gruppen, welche in der Kette anstelle eines Sauerstoffatomes, oder auch zusätzlich hierzu, eine Carboxylgruppe enthalten. Solche Gruppen sind beispielsweise Alkoxycarbonyl, Alkanoyloxy, Alkoxycarbonylalkyl oder Alkanoyloxyalkyl, Alkenoyloxy, Alkenyloxycarbonyl und dergleichen. Diese Gruppen können einfach oder mehrfach mit Halogen, insbesondere mit Fluor, substituiert sein. Solche Gruppen sind beispielsweise Acetoxy, Propanoyloxy, Butanoyloxy, Pentanoyloxy, Hexanoyloxy, Aethoxycarbonyl, Propyloxycarbonyl, Butyloxycarbonyl, Pentyloxycarbonyl, Hexyloxycarbonyl, 2E-Butenoyloxy, Pentenoyloxy, Hexenoyloxy, 2E-Butenoyloxy, 2E-Pentenoyloxy, 2E-Hexenoyloxy, 2E-Heptenoyloxy, 2E-Octenoyloxy, 2E-Nonenoyloxy, 2E-Decenoyloxy, 2E-Undecenoyloxy, 2E-Dodecenoyloxy, Allyloxycarbonyl, 2-Butenyloxycarbonyl, 3-Butenyloxycarbonyl, 2-Pentenyloxycarbonyl, 3-Pentenyloxycarbonyl, 4-Pentenyloxycarbonyl und dergleichen. Bevorzugt sind hier jedoch Gruppen mit l bis 7, bzw. 2 bis 7 Kohlenstoffatomen.

Der Ausdruck "unsubstituiertes oder einfach oder mehrfach Halogen substituiertes 1,4-Phenylen" umfasst im Rahmen der vorliegenden Erfindung 1,4-Phenylen, 2-Fluor-l,4-phenylen, 3-Fluor-l,4-phenylen, 2,3-Difluor-1,4-phenylen und dergleichen, insbesondere jedoch 1,4-Phenylen oder 2,3-Difluor-l,4-phenylen.

Der Ausdruck "Halogen" umfasst im Rahmen der vorliegenden Erfindung Fluor, Chlor, Brom und Jod, insbesondere jedoch Fluor.

Bevorzugte Verbindungen der allgemeinen Formel I sind Verbindungen der Formeln: 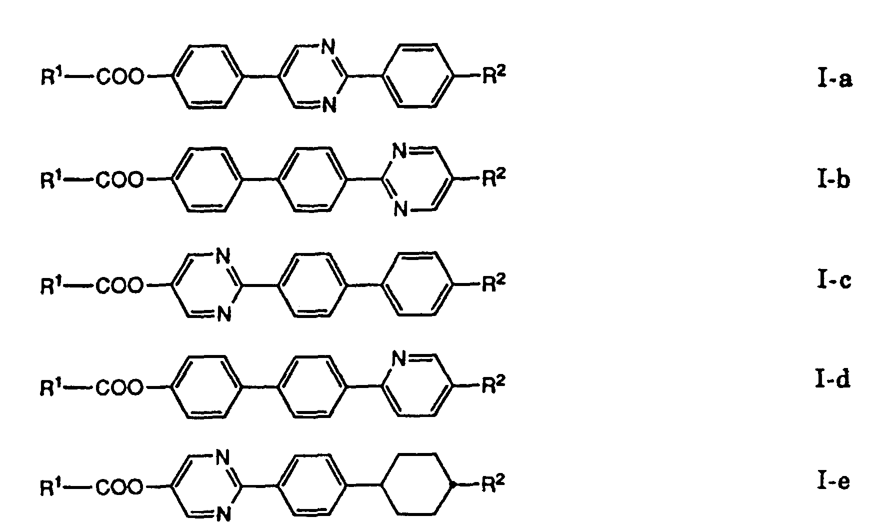 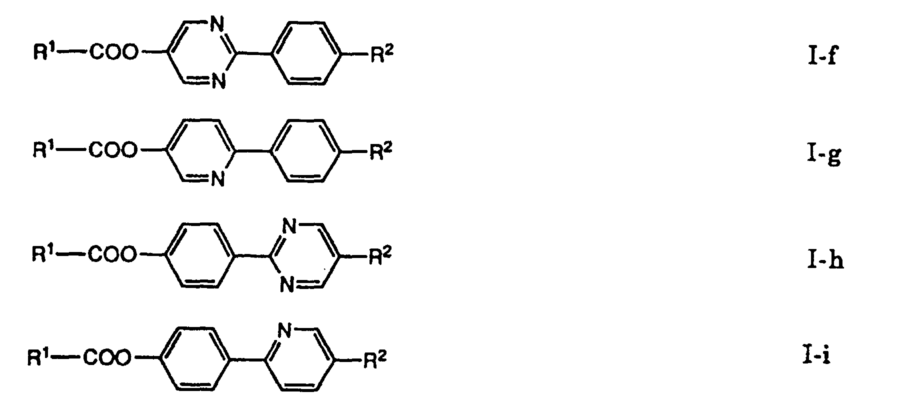 worin R¹ und R² die obigen Bedeutungen haben.

Die erfindungsgemässen Verbindungen der allgemeinen Formel I sind synthetisch leicht zugänglich und können in an sich bekannter Weise aus Phenolen bzw. 5-Hydroxypyridinen oder 5-Hydroxypyrimidinen und einer Säure hergestellt werden. Die Veresterung kann beispielsweise in Gegenwart von N,N'-Dicyclohexylcarbodiimid und 4-(Dimethylamino)pyridin in Dichlormethan oder einem anderen geeigneten Lösungsmittel, wie z.B. Chloroform, erfolgen. Die Ausgangsmaterialien sind bekannt und z.T. im Handel erhältlich, oder sie können, wie z.B. in den auf den Seiten 5 und 6 der EP-A2 0 546 298 dargestellten Schemata l bis 3 angegeben, hergestellt werden.

Die Verbindungen der Formel I können in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere bekannte flüssigkristalline Verbindungen sein. Es muss jedoch jeweils mindestens eine chirale Komponente im Gemisch enthalten sein. Sofern nicht bereits eine der verwendeten Komponenten chiral ist, muss demnach ein chiraler Dotierstoff zugesetzt werden.

Derartige Flüssigkristallkomponenten sind vorzugsweise achirale Verbindungen der Formeln 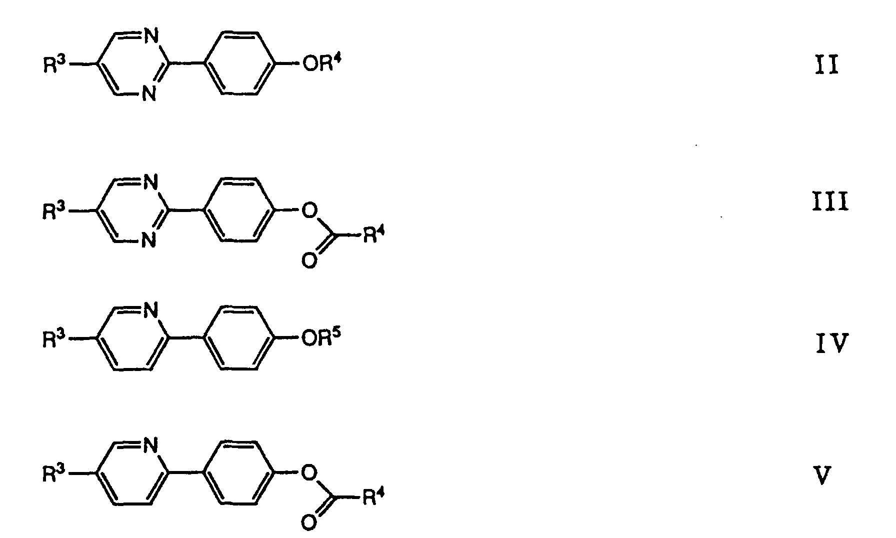 bzw. chirale Dotierstoffe der allgemeinen Formeln 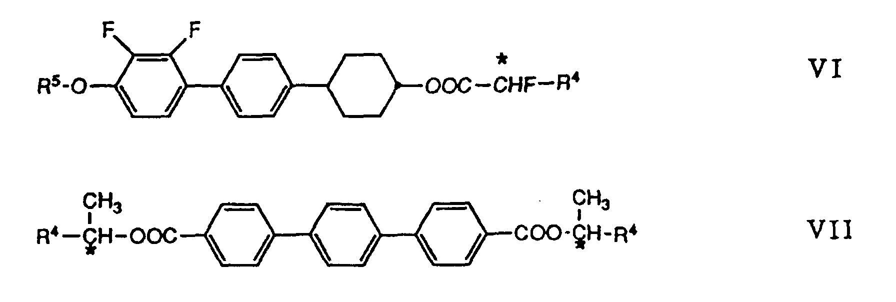 worin R³ Alkyl oder Alkoxy darstellt, R⁴ Alkyl bedeutet, und R⁵ Alkyl oder Alkenyl bedeutet.

Der Ausdruck "Alkyl" im Zusammenhang mit den Verbindungen der Formeln II bis VII umfasst unverzweigte oder verzweigte Alkylgruppen mit l bis 12 Kohlenstoffatomen, vorzugsweise unverzweigte Alkylgruppen mit 6 bis 12 Kohlenstoffatomen, wie Hexyl, Heptyl, Octyl, Nonyl, Decyl, Undecyl, Dodecyl.

Der Ausdruck "Alkoxy" umfasst Aethergruppen in denen der Alkylrest wie vorhergehend definiert ist.

Der Ausdruck "Alkenyl" umfasst im Zusammenhang mit den Verbindungen der Formeln II-VII Alkenylgruppen mit 2 bis 15 Kohlenstoffatomen, wie 2E-Alkenyl, 3Z-Alkenyl, 4E-Alkenyl und Alkenyl mit endständiger Doppelbindung. Die Ausdrücke "2E-Alkenyl", "3Z-Alkenyl" und 4E-Alkenyl" umfassen vorzugsweise unverzweigte Alkenylgruppen mit 3 bis 9, 4 bis 9 bzw. 5 bis 9 Kohlenstoffatomen, in welchen die Doppelbindung in 2, 3 bzw. 4 Stellung steht, wobei E und Z die Konfiguration der Doppelbindung bezeichnen. Solche Gruppen sind beispielsweise 2E-Butenyl, 2E-Pentenyl, 2E-Hexenyl, 2E-Heptenyl, 2E-Octenyl, 2E-Nonenyl, 3-Butenyl, 3Z-Pentenyl, 3Z-Hexenyl, 3Z-Heptenyl, 3Z-Octenyl, 3Z-Nonenyl, 4-Pentenyl, 4E-Hexenyl, 4E-Heptenyl, 4E-Octenyl, 4E-Nonenyl, 5-Hexenyl, 6-Heptenyl, 7-Octenyl, 8-Nonenyl und dergleichen.

Aufgrund der guten Löslichkeit der Verbindungen der Formel I und aufgrund ihrer guten Mischbarkeit untereinander kann der Anteil an Verbindungen der Formel I in den erfindungsgemässen Gemischen relativ hoch sein und bis etwa 85 Gew.% betragen. Bevorzugt wird im allgemeinen ein Anteil von etwa 1-50, insbesondere 5-30 Gew.% an Verbindungen der Formel I.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. In den Beispielen bedeutet C eine kristalline, N eine nematische, Ch eine chiral nematische, S eine smektische und I die isotrope Phase.

### Beispiel l

Zu einer Lösung von 1,0 g 4-(2-[4-Pentylphenyl]-5-pyrimidinyl)phenol, 0,4 g Caprinsäure und 0,05 g 4-(Dimethylamino)pyridin in 30 ml Dichlormethan wird unter Rühren innert 5 Minuten 0,8 g N,N'-Dicyclohexyl-carbodiimid gegeben. Das Reaktionsgemisch wird über Nacht weiter gerührt, dann filtriert, das Filtrat mit gesättigter Natriumbicarbonat-Lösung und mit Wasser gewaschen und eingeengt. Chromatographie des Rückstandes an Kieselgel mit Hexan/Aethylacetat (Vol. 4:1) und zweimaliges Umkristallisieren der gemäss Dünnschichtchromatographie reinen Fraktionen ergibt 1,0 g 4-(2-[4-Pentylphenyl]-5-pyrimidinyl)phenylhexanoat; Smp.(C-S_{C}) 124°C, Phasenübergang(S_{C}-S_{A}) 185°C, Klp.(S_{A}-I) 224°C.

Das als Ausgangsmaterial verwendete 4-(2-[4-Pentylphenyl]-5-pyrimidinylphenol wird wie folgt hergestellt:
a) Eine Lösung von 30 g 4-Hydroxybenzaldehyd und 46 g Isopropylbromid in 300 ml N,N-Dimethylformamid wird mit 104 g fein pulverisiertem Kaliumcarbonat versetzt und das Gemisch über Nacht bei 58°C gerührt. Die Suspension wird genutscht und das Filtrat im Vakuum eingeengt. Eine Lösung des Rückstands in 100 ml Diäthyläther wird zweimal mit je 100 ml Wasser gewaschen, über Natriumsulfat getrocknet und eingeengt. Chromatographie des Rohproduktes an Kieselgel mit Hexan/Aethylacetat (Vol. 9:1) ergibt 40 g 4-(Isopropyloxy)benzaldehyd.
b) Eine Suspension von 140 g Methoxymethyl-triphenylphosphoniumchiorid in 400 ml tert.-Butylmethyläther wird unter Stickstoffbegasung bei 0°C innert 5 Minuten mit 45 g Kalium-tert.-butylat versetzt und noch 10 Minuten gerührt. Sodann wird bei 0°C innert 30 Minuten eine Lösung von 41 g 4-(Isopropyloxy)-benzaldehyd in 100 ml tert.-Butylmethyläther zugetropft. Das Reaktionsgemisch wird noch 16 Stunden bei Raumtemperatur gerührt und dann auf 750 ml 0,8N Natriumhydrogencarbonat-Lösung gegossen. Die wässrige Phase wird abgetrennt und mit Diäthyläther nachextrahiert. Die organische Phase wird mit Wasser neutral gewaschen, über Magnesiumsulfat getrocknet und eingeengt. Der Rückstand wird in 800 ml Hexan aufgeschlämmt. Nach Abkühlen auf 0°C wird das Triphenylphosphinoxid abfiltriert und das Filtrat eingeengt. Chromatographie des Rückstandes an Kieselgel mit Hexan/Aethylacetat (Vol. 9.1) ergibt 45 g reines l-Methoxy-2-(4-[Isopropoxy]phenyl) äthen.
c) Unter Stickstoffatmosphäre werden bei 0°C zu 273 ml Orthoameisensäuretrimethylester zuerst 10 ml Bortrifluoriddiäthylätherat zugegeben und anschliessend 48 g l-Methoxy-2-(4-[isopropoxy]phenyl)äthen zugetropft. Das Reaktionsgemisch wird noch 4 Stunden bei 0°C gerührt und dann mit 6 ml Triäthanolamin neutralisiert und eingeengt. Der Rückstand wird in 500 ml Diäthyläther aufgenommen und die Lösung mit 100 ml gesättigter Natriumbicarbonat-Lösung und zweimal mit je 150 ml Wasser gewaschen, über Magnesiumsulfat getrocknet, filtriert und eingeengt. Chromatographie des Rückstandes an Kieselgel mit Hexan/Aethylacetat (Vol. 9:1) und Destillation bei 135-137°C (l mmHg) ergibt 44 g (4-[Isopropoxy]phenyl)malonaldehyd-tetramethylacetal.
d) Unter Stickstoffatmosphäre wird ein Gemisch aus 20,9 g (4-[Isopropyloxy]phenyl)malonaldehyd-tetramethylacetal 5,0 g p-Toluolsulfonsäuremonohydrat und 1,5 ml Wasser 2 Stunden auf 70-80°C erhitzt, dann mit 0,3 g Natriumbicarbonat versetzt, noch 5 Minuten gerührt und flitriert. Der Filterrückstand wird mit Methanol nachgewaschen und das Filtrat mit dem entstandenen rohen 2-(Methoxymethyliden)-2-(4-[isopropoxy]phenyl)acetaldehyd sofort in der nächsten Stufe eingesetzt.
e) Eine aus 14,7 ml Methanol und 2,5 g Natrium frisch hergestellte Natriummethylat-Lösung wird unter Stickstoffatmosphäre zu einem Gemisch aus 15,0 g p-Pentylbenzamidinhydrochlorid, 75 ml Methanol und der obigen methanolischen Lösung von 2-(Methoxymethyliden)-2-(4-[isopropyloxy]phenyl)acetaldehyd zugetropft. Das Reaktionsgemisch wird über Nacht gerührt und anschliessend mit konzentrierter Salzsäure auf pH 3-4 gestellt. Das ausgefallene 5-(4-Isopropyloxyphenyl)-2-(4-pentylphenyl)-pyrimidin wird abfiltriert, mit Wasser und mit Methanol gewaschen und im Vakuum getrocknet. Chromatographie des Rückstandes an Kieselgel mit Dichlormethan/Hexan (Vol. 1:1) ergibt 6,6 g reines Produkt.
f) Eine Lösung von 5,5 g Bortribromid in 50 ml absolutem Dichlormethan wird bei 0°C tropfenweise mit einer Lösung von 6,5 g 5-(4-Isopropyloxy-phenyl)-2-(4-pentylphenyl)pyrimidin in 50 ml absolutem Dichlormethan versetzt. Das Gemisch wird 24 Stunden gerührt und dann auf Eiswasser gegossen. Die organische Phase wird abgetrennt und die wässrige Phase dreimal mit je 50 ml Dichlormethan nachextrahiert. Die vereinigten organischen Phasen werden mit 50 ml 2N Natriumcarbonat-Lösung und mehrmals mit Wasser gewaschen, über Magnesiumsulfat getrocknet und eingeengt. Chromatographie des Rückstandes an Kieselgel mit Dichlormethan/Methanol (Vol. 99:1) und Umkristallisation aus Hexan ergibt 5,2 g 4-(2-[4-Pentylphenyl]-5-pyrimidinyl)phenol mit Smp. 152-153°C.

In analoger Weise können folgende Verbindungen hergestellt werden:
- 4-(2-[4-Propylphenyl]-5-pyrimidinyl)phenyl-acetat;
- 4-(2-[4-Propylphenyl]-5-pyrimidinyl)phenyl-propionat;
- 4-(2-[4-Propylphenyl]-5-pyrimidinyl)phenyl-butanoat;
- 4-(2-[4-Propylphenyl]-5-pyrimidinyl)phenyl-pentanoat;
- 4-(2-[4-Propylphenyl]-5-pyrimidinyl)phenyl-hexanoat;
- 4-(2-[4-Propylphenyl]-5-pyrimidinyl)phenyl-heptanoat;
- 4-(2-[4-Propylphenyl]-5-pyrimidinyl)phenyl-acetat,
   Smp.(C-S_{C}) 169°C, S_{C}-S_{A} 137°C, S_{A}-N 179°C, Klp.(N-I)222°C;
- 4-(2-[4-Propylphenyl]-5-pyrimidinyl)phenyl-propionat,
   Smp.(C-S_{C})150°C, S_{C}-S_{A} 166°C, S_{A}-N 217°C, Klp.(N-I)230°C;
- 4-(2-[4-Propylphenyl]-5-pyrimidinyl)phenyl-butanoat,
   Smp.(C-S_{C})143°C,S_{C}-S_{A} 170°C, SA-N 225°C, Klp.(N-I)229°C;
- 4-(2-[4-Propylphenyl]-5-pyrimidinyl)phenyl-pentanoat,
   Smp.(C-S_{C})139°C, S_{C}-S_{A} 184°C, Klp.(S_{A}-I)224°C;
- 4-(2-[4-Propylphenyl]-5-pyrimidinyl)phenyl-hexanoat,
   Smp.(C-S₃)124°C,S₃-S_{C} 132°C, S_{C}-S_{A} 184°C, Klp.(S_{A}-I)224°C;
- 4-(2-[4-Propylphenyl]-5-pyrimidinyl)phenyl-heptanoat;
- 4-(2-[4-Propylphenyl]-5-pyrimidinyl)phenyl-octanoat,
   Smp.(C-S₃)110°C, S₃⁻S_{C} 134°C, S_{C}-S_{A} 197°C, Klp.(S_{A}-I)217°C;
- 4-(2-[4-Heptylphenyl]-5-pyrimidinyl)phenyl-acetat;
- 4-(2-[4-Heptylphenyl]-5-pyrimidinyl)phenyl-propionat;
- 4-(2-[4-Heptylphenyl]-5-pyrimidinyl)phenyl-butanoat;
- 4-(2-[4-Heptylphenyl]-5-pyrimidinyl)phenyl-pentanoat;
- 4-(2-[4-Heptylphenyl]-5-pyrimidinyl)phenyl-hexanoat;
- 4-(2-[4-Heptylphenyl]-5-pyrimidinyl)phenyl-heptanoat.

### Beispiel 2

2,0 g Caprinsäure, 5,0 g 4-(4-[5-Decyl-2-pyrimidinyl]phenyl)phenol, 3,0 g N,N'-Dicyclohexylcarbodiimid, 0,4 g 4-(Dimethylamino)pyridin und 100 ml Dichlormethan werden in analoger Weise zu Beispiel l zu 5 g 4-(4-[5-Decyl-2-pyrimidinyl]-4'-biphenyl)-hexanoat mit Smp.(C-S₄) 34°C, Phasenübergang(S₄-S₃) 53°C, Phasenübergang(S₃-S₂) 66°C, Phasenübergang(S₂- S_{C}) 124°C, Phasenübergang(S_{C}-N) 169°C und Klp.(N-I) 180°C umgesetzt.

Das als Ausgangsmaterial verwendete 4-(4-[5-Decyl-2-pyrimidinyl]-phenyl)phenol wird wie folgt hergestellt:
a) Eine Lösung von 30 g 4-Cyano-4'-(pentyloxy)biphenyl, 23 ml Aethylalkohol und 200 ml Toluol wird bei 0°C vorgelegt und während 6 Stunden Chlorwasserstoff eingeleitet. Das Reaktionsgemisch wird noch 48 Stunden bei Raumtemperatur weitergerührt, dann eingeengt und unter Vakuum getrocknet. Dies ergibt 39 g Aethyl-4-[4-(pentyloxy)phenyl]benzimidathydrochlorid.
b) Ein Gemisch von 39 g Aethyl-4-[4-(pentyloxy)phenyl]-benzimidat-hydrochlorid und 100 ml Aethylalkohol wird bei Raumtemperatur und unter Stickstoffbegasung vorgelegt, dann mit 82,5 ml gesättigter äthanolischer Ammoniak-Lösung versetzt und über Nacht gerührt. Das Reaktionsgemisch wird eingeengt, unter Vakuum getrocknet, in Diäthyläther suspendiert, 2 Stunden bei Raumtemperatur gerührt, auf 0°C abgekühlt und abgenutscht. Die weissen Kristalle werden mit Diäthyläther gewaschen und dann unter Vakuum getrocknet. Dies ergibt 35,7 g 4-(4-[Pentyloxy]phenyl)-benzamidin-hydrochlorid.
c) 44,5 g 2-(Decyl)malonaldehyd-tetramethylacetal, 17 g p-Toluolsulfonsäuremonohydrat und 2 ml Wasser werden in analoger Weise zu Beispiel l(d) zu 2-(Methoxymethyliden)dodecanal umgesetzt.
d) 2-(Methoxymethyliden)dodecanal in 10 ml Methanol, 20,6 ml 30%iger Natriummethylat-Lösung und 35,7 g 4-(4-[Pentyloxy]phenyl)benzamidin-hydrochlorid werden in analoger Weise zu Beispiel l(e) zu 21,3 g 5-Decyl-2-(4-[pentyloxy]-4'-biphenyl) pyrimidin umgesetzt.
e) 21,3 g 5-Decyl-2-(4-[pentyloxy]-4'-biphenyl)pyrimidin, 17,7 g Bortribromid und 100 ml Dichlormethan werden in analoger Weise zu Beispiel l(f) zu 12,5 g 4-(4-[5-Decyl-2-pyrimidinyl]phenyl)phenol umgesetzt.

In analoger Weise können folgende Verbindungen hergestellt werden:
- 4-[5-Propyl-2-pyrimidinyl]-4'-biphenyl-acetat;
- 4-[5-Propyl-2-pyrimidinyl]-4'-biphenyl-propanoat;
- 4-[5-Propyl-2-pyrimidinyl]-4'-biphenyl-butanoat;
- 4-[5-Propyl-2-pyrimidinyl]-4'-biphenyl-pentanoat;
- 4-[5-Propyl-2-pyrimidinyl]-4'-biphenyl-hexanoat;
- 4-[5-Propyl-2-pyrimidinyl]-4'-biphenyl-heptanoat;
- 4-[5-Pentyl-2-pyrimidinyl]-4'-biphenyl-acetat;
- 4-[5-Pentyl-2-pyrimidinyl]-4'-biphenyl-propanoat;
- 4-[5-Pentyl-2-pyrimidinyl]-4'-biphenyl-butanoat;
- 4-[5-Pentyl-2-pyrimidinyl]-4'-biphenyl-pentanoat;
- 4-[5-Pentyl-2-pyrimidinyl]-4'-biphenyl-hexanoat;
- 4-[5-Pentyl-2-pyrimidinyl]-4'-biphenyl-heptanoat;
- 4-[5-Heptyl-2-pyrimidinyl]-4'-biphenyl-acetat;
- 4-[5-Heptyl-2-pyrimidinyl]-4'-biphenyl-propanoat;
- 4-[5-Heptyl-2-pyrimidinyl]-4'-biphenyl-butanoat;
- 4-[5-Heptyl-2-pyrimidinyl]-4'-biphenyl-pentanoat;
- 4-[5-Heptyl-2-pyrimidinyl]-4'-biphenyl-hexanoat;
- 4-[5-Heptyl-2-pyrimidinyl]-4'-biphenyl-heptanoat;
- 4-[5-Octyl-2-pyrimidinyl]-4'-biphenyl-acetat;
- 4-[5-Octyl-2-pyrimidinyl]-4'-biphenyl-propanoat;
- 4-[5-Octyl-2-pyrimidinyl]-4'-biphenyl-butanoat;
- 4-[5-Octyl-2-pyrimidinyl]-4'-biphenyl-pentanoat;
- 4-[5-Octyl-2-pyrimidinyl]-4'-biphenyl-hexanoat;
- 4-[5-Octyl-2-pyrimidinyl]-4'-biphenyl-heptanoat;
- 4-[5-Decyl-2-pyrimidinyl]-4'-biphenyl-acetat;
- 4-[5-Decyl-2-pyrimidinyl]-4'-biphenyl-propanoat;
- 4-[5-Decyl-2-pyrimidinyl]-4'-biphenyl-butanoat;
- 4-[5-Decyl-2-pyrimidinyl]-4'-biphenyl-pentanoat;
- 4-[5-Decyl-2-pyrimidinyl]-4'-biphenyl-heptanoat;
- l-(5-Pentyl-2-pyrimidinyl)-4-phenyl-(2E)-butenoat;
- l-(5-Pentyl-2-pyrimidinyl)-4-phenyl-(2E)-pentenoat;
- l-(5-Pentyl-2-pyrimidinyl)-4-phenyl-(2E)-hexenoat;
- l-(5-Pentyl-2-pyrimidinyl)-4-phenyl-(2E)-heptenoat;
- l-(5-Pentyl-2-pyrimidinyl)-4-phenyl-(2E)-octenoat;
- l-(5-Pentyl-2-pyrimidinyl)-4-phenyl-(2E)-nonenoat;
- l-(5-Pentyl-2-pyrimidinyl)-4-phenyl-(2E)-decenoat;
- l-(5-Pentyl-2-pyrimidinyl)-4-phenyl-(2E)-undecenoat;
- l-(5-Pentyl-2-pyrimidinyl)-4-phenyl-(2E)-dodecenoat;
- l-(5-Hexyl-2-pyrimidinyl)-4-phenyl-(2E)-butenoat;
- l-(5-Hexyl-2-pyrimidinyl)-4-phenyl-(2E)-pentenoat;
- l-(5-Hexyl-2-pyrimidinyl)-4-phenyl-(2E)-hexenoat;
- l-(5-Hexyl-2-pyrimidinyl)-4-phenyl-(2E)-heptenoat;
- l-(5-Hexyl-2-pyrimidinyl)-4-phenyl-(2E)-octenoat;
- l-(5-Hexyl-2-pyrimidinyl)-4-phenyl-(2E)-nonenoat
- l-(5-Hexyl-2-pyrimidinyl)-4-phenyl-(2E)-decenoat;
- l-(5-Hexyl-2-pyrimidinyl)-4-phenyl-(2E)-undecenoat;
- l-(5-Hexyl-2-pyrimidinyl)-4-phenyl-(2E)-dodecenoat;
- l-(5-Hexyl-2-pyrimidinyl)-4-phenyl-(2E)-butenoat,
   Smp.(C-I) 97°C, (N-I) 87°C;
- l-(5-Heptyl-2-pyrimidinyl)-4-phenyl-(2E)-pentenoat,
   Smp.(C-I) 104°C, (N-I) 69°C;
- l-(5-Heptyl-2-pyrimidinyl)-4-phenyl-(2E)-hexenoat,
   Smp.(C-N) 70°C, Klp.(N-I) 82°C;
- l-(5-Heptyl-2-pyrimidinyl)-4-phenyl-(2E)-heptenoat,
   Smp.(C-N) 48°C, Klp.(N-I) 72°C;
- l-(5-Heptyl-2-pyrimidinyl)-4-phenyl-(2E)-octenoat,
   Smp.(C-N) 50°C, Klp.(N-I) 80°C;
- l-(5-Heptyl-2-pyrimidinyl)-4-phenyl-(2E)-nonenoat;
- l-(5-Heptyl-2-pyrimidinyl)-4-phenyl-(2E)-decenoat,
   Smp.(C-N) 59°C Klp.(N-I) 80°C;
- l-(5-Heptyl-2-pyrimidinyl)-4-phenyl-(2E)-undecenoat;
- l-(5-Heptyl-2-pyrimidinyl)-4-phenyl-(2E)-dodecenoat,
   Smp.(C-N) 52°C, Klp.(N-I) 80°C;
- l-(5-Octyl-2-pyrimidinyl)-4-phenyl-(2E)-butenoat,
   Smp.(C-N) 65°C, Klp.(N-I)80°C;
- l-(5-Octyl-2-pyrimidinyl)-4-phenyl-(2E)-pentenoat,
   Smp.(C-I) 83°C, (N-I) 64°C;
- l-(5-Octyl-2-pyrimidinyl)-4-phenyl-(2E)-hexenoat,
   Smp.(C-N) 70°C, Klp.(N-I) 76°C;
- l-(5-Octyl-2-pyrimidinyl)-4-phenyl-(2E)-heptenoat,
   Smp.(C-N) 54°C, Klp.(N-I) 66°C;
- l-(5-Octyl-2-pyrimidinyl)-4-phenyl-(2E)-octenoat,
   Smp.(C-N) 46°C, Klp.(N-I) 75°C;
- l-(5-Octyl-2-pyrimidinyl)-4-phenyl-(2E)-nonenoat;
- l-(5-Octyl-2-pyrimidinyl)-4-phenyl-(2E)-decenoat,
   Smp.(C-N) 41°C, Klp.(N-I) 76°C;
- l-(5-Octyl-2-pyrimidinyl)-4-phenyl-(2E)-undecenoat;
- l-(5-Octyl-2-pyrimidinyl)-4-phenyl-(2E)-dodecenoat,
   Smp.(C-N) 52°C, Klp.(N-I) 78°C;
- l-(5-Nonyl-2-pyrimidinyl)-4-phenyl-(2E)-butenoat,
   Smp.(C-N) 80°C, Klp.(N-I) 89°C;
- l-(5-Nonyl-2-pyrimidinyl)-4-phenyl-(2E)-pentenoat,
   Smp.(C-I) 89°C,(N-I) 70°C;
- l-(5-Nonyl-2-pyrimidinyl)-4-phenyl-(2E)-hexenoat,
   Smp.(C-N) 68°C, Klp.(N-I) 81°C;
- l-(5-Nonyl-2-pyrimidinyl)-4-phenyl-(2E)-heptenoat,
   Smp.(C-N) 47°C Klp.(N-I) 70°C;
- l-(5-Nonyl-2-pyrimidinyl)-4-phenyl-(2E)-octenoat,
   Smp.(C-N) 49°C, Klp.(N-I) 79°C;
- l-(5-Nonyl-2-pyrimidinyl)-4-phenyl-(2E)-decenoat,
   Smp.(C-N) 50°C, Klp.(N-I) 81°C;
- l-(5-Nonyl-2-pyrimidinyl)-4-phenyl-(2E)-undecenoat;
- l-(5-Nonyl-2-pyrimidinyl)-4-phenyl-(2E)-dodecenoat,
   Smp.(C-N) 60°C, Klp.(N-I) 82°C.

### Beispiel 3

0,2 g Caprinsäure, 0,4 g 2-[4-Pentyl-4'-biphenyl]-5-hydroxypyrimidin, 0,3 g N,N'-Dicyclohexylcarbodiimid, 0,04 g 4-(Dimethylamino)pyridin und 50 ml Dichlormethan werden in analoger Weise zu Beispiel l zu 0,4 g 2-[4-Pentyl-4'-biphenyl]-5-([hexanoyl]oxy)pyrimidin umgesetzt.

Das als Ausgangsmaterial verwendete 2-[4-Pentyl-4'-biphenyl]-5-hydroxypyrimidin wird wie folgt hergestellt:
a) Eine Lösung aus 50 ml Methanol und 2,6 g Natrium frisch hergestellte Natriummethylat-Lösung wird unter Stickstoffbegasung mit 15 g 4-(4-Pentylphenyl)benzamidin-hydrochlorid und 13,5 g l,3-bis(Dimethylamino)-2-äthoxytrimethinium-perchlorat (Cell. Czech. Chem. Commun. 38, 1168, 1973) in 80 ml Methanol versetzt. Das Reaktionsgemisch wird 5 Stunden unter leichtem Rückfluss erwärmt, dann auf 100 ml Wasser gegossen, mit konzentrierter Salzsäure neutralisiert und dann dreimal mit je 100 ml Diäthyläther extrahiert. Die vereinigten organischen Phasen werden mit 100 ml konzentrierter Kochsalz-Lösung, 100 ml konzentrierter Kaliumcarbonat-Lösung und dann nochmals mit 100 ml konzentrierter Kochsalz-Lösung gewaschen, über Magnesiumsulfat getrocknet, filtriert und anschliessend eingeengt. Der Rückstand wird an Kieselgel mit Hexan/Aethyl-acetat (Vol. 9:1) chromatographiert und dann aus Aethylalkohol umkristallisiert. Dies ergibt 14,7 g reines 2-(4-Pentyl-4'-biphenyl)-5-(äthoxy)pyrimidin.
b) Ein Gemisch von 14,7 g 2-[4-Pentyl-4'-biphenyl]-5-(äthoxy)pyrimidin, 17 g Natriumhydroxid und 120 ml Diäthylenglykol wird 8 Stunden bei 180°C erwärmt. Das Reaktionsgemisch wird auf 100 ml Wasser gegossen, mit konzentrierter Salzsäure angesäuert, dann dreimal mit je 50 ml Diäthyläther extrahiert. Die vereinigten organischen Phasen werden zweimal mit 100 ml konzentrierter Kochsalz-Lösung gewaschen, über Magnesiumsulfat getrocknet, filtriert und anschliessend eingeengt. Chromatographie des Rückstandes an Kieselgel mit Hexan/Aethylacetat (Vol. 7:3) und Umkristallisation aus Aethylalkohol ergibt 11 g 2-[4-Pentyl-4'-biphenyl]-5-hydroxypyrimidin.

In analoger Weise können folgende Verbindungen hergestellt werden:
- 2-[4-Propyl-4'-biphenyl]-5-(acetoxy)pyrimidin;
- 2-[4-Propyl-4'-biphenyl]-5-([propanoy]oxy)pyrimidin;
- 2-[4-Propyl-4'-biphenyl]-5-([butanoyl]oxy)pyrimidin;
- 2-[4-Propyl-4'-biphenyl]-5-([pentanoyl]oxy)pyrimidin;
- 2-[4-Propyl-4'-biphenyl]-5-([hexanoyl]oxy)pyrimidin;
- 2-[4-Propyl-4'-biphenyl]-5-([heptanoyl]oxy)pyrimidin;
- 2-[4-Propyl-4'-biphenyl]-5-([octanoyl]oxy)pyrimidin;
- 2-[4-Propyl-4'-biphenyl]-5-([nonanoyl]oxy)pyrimidin;
- 2-[4-Propyl-4'-biphenyl]-5-([decanoyl]oxy)pyrimidin;
- 2-[4-Propyl-4'-biphenyl]-5-([(E)-2-pentenoyl]oxy)pyrimidin;
- 2-[4-Propyl-4'-biphenyl]-5-([(Z)-3-pentenoyl]oxy)pyrimidin;
- 2-[4-Propyl-4'-biphenyl]-5-([4-pentenoyl]oxy)pyrimidin;
- 2-[4-Pentyl-4'-biphenyl]-5-(acetoxy)pyrimidin;
- 2-[4-Pentyl-4'-biphenyl]-5-([propanoyl]oxy)pyrimidin;
- 2-[4-Pentyl-4'-biphenyl]-5-([butanoyl]oxy)pyrimidin;
- 2-[4-Pentyl-4'-biphenyl]-5-([pentanoyl]oxy)pyrimidin;
- 2-[4-Pentyl-4'-biphenyl]-5-([hexanoyl]oxy)pyrimidin;
- 2-[4-Pentyl-4'-biphenyl]-5-([heptanoyl]oxy)pyrimidin;
- 2-[4-Pentyl-4'-biphenyl]-5-([octanoyl]oxy)pyrimidin;
- 2-[4-Pentyl-4'-biphenyl]-5-([nonanoyl]oxy)pyrimidin;
- 2-[4-Pentyl-4'-biphenyl]-5-([decanoyl]oxy)pyrimidin;
- 2-[4-Pentyl-4'-biphenyl]-5-([(E)-2-pentenoyl]oxy)pyrimidin;
- 2-[4-Pentyl-4'-biphenyl]-5-([(Z)-3-pentenoyl]oxy)pyrimidin;
- 2-[4-Pentyl-4'-biphenyl]-5-([4-pentenoyl]oxy)pyrimidin;
- 2-[4-Heptylphenyl]-5-[(2E-butenoyl)oxy]pyrimidin;
- 2-[4-Heptylphenyl]-5-[(2E-pentenoyl)oxy]pyrimidin;
- 2-[4-Heptylphenyl]-5-[(2E-hexenoyl)oxy]pyrimidin;
- 2-{4-Heptylphenyl]-5-[(2E-heptenoyl)oxy]pyrimidin;
- 2-[4-Heptylphenyl]-5-[(2E-octenoyl)oxy]pyrimidin,
   Smp.(C-N) 80°C, Klp.(N-I) 86°C;
- 2-[4-Heptylphenyl]-5-[(2E-nonenoyl)oxy]pyrimidin;
- 2-[4-Heptylphenyl]-5-[(2E-decenoyl)oxy]pyrimidin;
- 2-[4-Heptylphenyl]-5-[(2E-undecenoyl)oxy]pyrimidin;
- 2-[4-Heptylphenyl]-5-[(2E-dodecenoyl)oxy]pyrimidin;
- 2-[4-Octylphenyl]-5-[(2E-butenoyl)oxy]pyrimidin;
- 2-[4-Octylphenyl]-5-[(2E-pentenoyl)oxy]pyrimidin;
- 2-[4-Octylphenyl]-5-[(2E-hexenoyl)oxy]pyrimidin;
- 2-[4-Octylphenyl]-5-[(2E-heptenoyl)oxy]pyrimidin;
- 2-[4-Octylphenyl]-5-[(2E-octenoyl)oxy]pyrimidin;
- 2-[4-Octylphenyl]-5-[(2E-nonenoyl)oxy]pyrimidin;
- 2-[4-Octylphenyl]-5-[(2E-decenoyl)oxy]pyrimidin;
- 2-[4-Octylphenyl]-5-[(2E-undecenoyl)oxy]pyrimidin;
- 2-[4-Octylphenyl]-5-[(2E-dodecenoyl)oxy]pyrimidin;
- 2-(4-[2-(4-Pentylphenyl)äthyl]phenyl-5-(acetoxy)pyrimidin;
- 2-(4-[2-(4-Pentylphenyl)äthyl]phenyl-5-([propanoyl]oxy)pyrimidin;
- 2-(4-[2-(4-Pentylphenyl)äthyl]phenyl-5-([butanoyl]oxy)pyrimidin;
- 2-(4-[2-(4-Pentylphenyl)äthyl]phenyl-5-([pentanoyl]oxy)pyrimidin;
- 2-(4-[2-(4-Pentylphenyl)äthyl]phenyl-5-([hexanoyl]oxy)pyrimidin;
- 2-(4-[2-(4-Pentylphenyl)äthyl]phenyl-5-([heptanoyl]oxy)pyrimidin;
- 2-(4-[2-(4-Pentylphenyl)äthyl]phenyl-5-([octanoyl]oxy)pyrimidin;
- 2-[4-(trans-4-Propylcyclohexyl)phenyl]-5-(acetoxy)pyrimidin;
- 2-[4-(trans-4-Propylcyclohexyl)phenyl]-5-([propanoyl]oxy)pyrimidin;
- 2-[4-(trans-4-Propylcyclohexyl)phenyl]-5-([butanoyl]oxy)pyrimidin;
- 2-[4-(trans-4-Propylcyclohexyl)phenyl]-5-([pentanoyl]oxy)pyrimidin;
- 2-[4-(trans-4-Propylcyclohexyl)phenyl]-5-([hexanoyl]oxy)pyrimidin;
- 2-[4-(trans-4-Propylcyclohexyl)phenyl]-5-([heptanoyl]oxy)pyrimidin;
- 2-[4-(trans-4-Propylcyclohexyl)phenyl]-5-([octanoyl]oxy)pyrimidin;
- 2-[4-(trans-4-Pentylcyclohexyl)phenyl]-5-(acetoxy)pyrimidin;
- 2-[4-(trans-4-Pentylcyclohexyl)phenyl]-5-([propanoyl]oxy)pyrimidin;
- 2-[4-(trans-4-Pentylcyclohexyl)phenyl]-5-([butanoyl]oxy)pyrimidin;
- 2-[4-(trans-4-Pentylcyclohexyl)phenyl]-5-([pentanoyl]oxy)pyrimidin;
- 2-[4-(trans-4-Pentylcyclohexyl)phenyl]-5-([hexanoyl]oxy)pyrimidin;
- 2-[4-(trans-4-Pentylcyclohexyl)phenyl]-5-([heptanoyl]oxy)pyrimidin,
   Smp.(C-N) 112°C, Klp.(N-I) 188°C;
- 2-(4-(trans-4-Pentylcyclohexyl)phenyl]-5-([octanoyl]oxy)pyrimidin,
   Smp.(C-N) 120°C, Klp. (N-I) 189°C;
- 2-[4-(trans-4-Pentylcyclohexyl)phenyl]-5-([nonanoyl]oxy)pyrimidin;
- 2-[4-(trans-4-Pentylcyclohexyl)phenyl]-5-([decanoyl]oxy)pyrimidin;
- 2-[4-(trans-4-Pentylcyclohexyl)phenyl]-5-([undecanoyl]oxy)pyrimidin,
   Smp.(C-N) 138°C, Klp.(N-I) 169°C;
- 2-[4-(trans-4-Pentylcyclohexyl)phenyl]-5-([dodecanoyl]oxy)pyrimidin,
   Smp.(C-S_{C}) 120°C, S_{C}-N 138°C, Klp.(N-I) 169°C;
- 2-[4-(trans-4-Heptylcyclohexyl)phenyl]-5-(acetoxy)pyrimidin;
- 2-[4-(trans-4-Heptylcyclohexyl)phenyl]-5-([propanoyl]oxy)pyrimidin;
- 2-[4-(trans-4-Heptylcyclohexyl)phenyl]-5-([butanoyl]oxy)pyrimidin;
- 2-[4-(trans-4-Heptylcyclohexyl)phenyl]-5-([pentanoyl]oxy)pyrimidin;
- 2-[4-(trans-4-Heptylcyclohexyl)phenyl]-5-([hexanoyl]oxy)pyrimidin;
- 2-[4-(trans-4-Heptylcyclohexyl)phenyl]-5-([heptanoyl]oxy)pyrimidin;
- 2-[4-(trans-4-Heptylcyclohexyl)phenyl]-5-([octanoyl]oxy)pyrimidin;
- 2-(4-[2-(trans-4-Pentylcyclohexyl)äthyl]phenyl-5-(acetoxy)pyrimidin;
- 2-(4-[2-(trans-4-Pentylcyclohexyl)äthyl]phenyl-5-([propanoyl]oxy)pyrimidin;
- 2-(4-[2-(trans-4-Pentylcyclohexyl)äthyl]phenyl-5-([butanoyl]oxy)pyrimidin;
- 2-(4-[2-(trans-4-Pentylcyclohexyl)äthyl]phenyl-5-([pentanoyl]oxy)pyrimidin;
- 2-(4-[2-(trans-4-Pentylcyclohexyl)äthyl]phenyl-5-([hexanoyl]oxy)pyrimidin;
- 2-(4-[2-(trans-4-Pentylcyclohexyl)äthyl]phenyl-5-([heptanoyl]oxy)pyrimidin;
- 2-(4-[2-(trans-4-Pentylcyclohexyl)äthyl]phenyl-5-([octanoyl]oxy)pyrimidin.

### Beispiel 4

0,25 g Caprinsäure, 1,0 g 4-(5-Pentyl-2-pyridinyl)-4'-hydroxybiphenyl, 0,8 g N,N-Dicyclohexylcarbodiimid, 0,04 g 4-(Dimethylamino)pyridin und 50 ml Dichlormethan werden in analoger Weise zu Beispiel 1 zu 4-(5-Pentyl-2-pyridinyl)-4'-biphenylhexanoat umgesetzt.

Das als Ausgangsmaterial verwendete 4-(5-Pentyl-2-pyridinyl)-4'-hydroxybiphenyl wird wie folgt hergestellt:
a) 20 g 4-Brom-4'-hydroxybiphenyl, 12 g Isopropylbromid, 45 g Kalium-carbonat und 200 ml N,N-Dimethylformamid werden in analoger Weise zu Beispiel 1(a) zu 18 g 4-Brom-4'-(isopropoxy)biphenyl umgesetzt.
b) Eine aus 0,8 g Magnesium, 10 g 4-Brom-4'-(isopropoxy)biphenyl und 50 ml Tetrahydrofuran bereitete Grignard-Reagens-Lösung wird bei 0-5°C innert 30 Minuten in ein Gemisch aus 7,5 g rohem 2-Chlor-5-[(tetrahydro-2-pyranyloxy)methyl]pyridin, 75 ml Tetrahydrofuran und 0,36 g 1,3-Bis-(diphenylphosphino)propannickel-(II)-chlorid getropft. Das Reaktionsgemisch wird 3 Stunden gerührt, über Nacht stehen gelassen, dann mit wässriger Natriumhydrogencarbonat-Lösung auf pH 8 gestellt und mit Diäthyläther verdünnt. Die organische Phase wird mit Wasser neutral gewaschen, über Natriumsulfat getrocknet und eingeengt. Chromatographische Reinigung des Rückstandes an Kieselgel mit Hexan/Aethylacetat (Vol. 4:1) liefert 8,8 g 2-[4-(Isopropoxy)biphenyl]-5-[(tetrahydro-2-pyranyloxy)methyl]pyridin.
c) Eine Lösung von 8,2 g 2-[4-(Isopropoxy)biphenyl]-5-[(tetrahydro-2-pyranyloxy)methyl]pyridin in 150 ml Tetrahydrofuran wird mit 22 ml 2N Salzsäure versetzt. Das Gemisch wird 6 Stunden bei 60°C gerührt, dann mit Diäthyläther verdünnt, zweimal mit je 50 ml gesättigter wässriger Natriumhydrogencarbonat-Lösung und dreimal mit je 75 ml Wasser gewaschen, über Natriumsulfat getrocknet und eingeengt. Es resultieren 7,2 g 6-[4-(Isopropoxy)biphenyl]-3-pyridincarbinol.
d) Eine Lösung von 7,2 g 6-[4-(Isopropoxy)biphenyl]-3-pyridincarbinol in 150 ml 1,2-Dichloräthan wird mit 9,6 g aktiviertem Mangan-(IV)-oxid versetzt. Die Suspension wird 4,5 Stunden zum Sieden erhitzt und dann filtriert. Einengen des Filtrates ergibt 5,0 g 6-[4-(Isopropoxy)biphenyl]-3-pyridincarboxaldehyd.
e) 6,0 g 6-[4-(Isopropoxy)biphenyl]-3-pyridincarboxaldehyd, 7,9 g Butyl-triphenylphosphoniumchlorid, 2,5 g Kalium-tert.-butylat und 100 ml tert.-Butyl-methyläther werden in analoger Weise zu Beispiel l(b) zu 5,7 g 2-[4-(Isopropoxy)-4'-biphenyl]-5-(pentenyl)pyridin als cis/trans-Gemisch umgesetzt.
f) 5,7 g 2-[4-(Isopropoxy)-4'-biphenyl]-5-(pentenyl)pyridin, 1 g Palladium auf Aktivkohle (10 w/w %) und 100 ml Aethylacetat werden in analoger Weise zu Beispiel 4(c) zu 5,3 g 2-[4-(Isopropoxy)-4'-biphenyl]-5-(pentyl)pyridin hydriert.
g) 5,3 g -[4-(Isopropoxy)-4'-biphenyl]-5-(pentyl)pyridin, 7,0 g Bortribromid in 100 ml Dichlormethan werden in analoger Weise zu Beispiel l(f) zu 3,7 g 4-(5-Pentyl-2-pyridinyl)-4'-hydroxybiphenyl umgesetzt.

In analoger Weise können folgende Verbindungen hergestellt werden:
- 4-[5-Pentyl-2-pyridinyl]-4'-biphenyl-acetat;
- 4-[5-Pentyl-2-pyridinyl]-4'-biphenyl-propionat;
- 4-[5-Pentyl-2-pyridinyl]-4'-biphenyl-butanoat;
- 4-[5-Pentyl-2-pyridinyl]-4'-biphenyl-pentanoat;
- 4-[5-Pentyl-2-pyridinyl]-4'-biphenyl-heptanoat;
- 4-[5-Pentyl-2-pyridinyl]-4'-biphenyl-octanoat;
- 4-[5-Pentyl-2-pyridinyl]-4'-biphenyl-decanoat;
- 4-[5-Pentyl-2-pyridinyl]-4'-biphenyl-undecanoat;
- 4-[5-Pentyl-2-pyridinyl]-4'-biphenyl-dodecanoat;
- 4-(5-Heptyl-2-pyridinyl)-4-biphenyl-acetat;
- 4-(5-Heptyl-2-pyridinyl)-4-biphenyl-propanoat;
- 4-(5-Heptyl-2-pyridinyl)-4-biphenyl-butanoat;
- 4-(5-Heptyl-2-pyridinyl)-4-biphenyl-pentanoat;
- 4-(5-Heptyl-2-pyridinyl)-4-biphenyl-hexanoat;
- 4-(5-Heptyl-2-pyridinyl)-4-biphenyl-heptanoat;
- 4-(5-Heptyl-2-pyridinyl)-4-biphenyl-octanoat;
- 4-(5-Heptyl-2-pyridinyl)-4-biphenyl-nonanoat;
- 4-(5-Heptyl-2-pyridinyl)-4-biphenyl-decanoat;
- 4-(5-Heptyl-2-pyridinyl)-4-biphenyl-undecanoat;
- 4-(5-Heptyl-2-pyridinyl)-4-biphenyl-dodecanoat;
- 1-(5-Hexyl-2-pyridinyl)-4-phenyl-(2E)-heptenoat;
- 1-(5-Hexyl-2-pyridinyl)-4-phenyl-(2E)-octenoat;
- 1-(5-Hexyl-2-pyridinyl)-4-phenyl-(2E)-nonenoat;
- 1-(5-Heptyl-2-pyridinyl)-4-phenyl-(2E)-butenoat,
   Smp.(C-N) 72°C, Klp.(N-I) 93°C;
- 1-(5-Heptyl-2-pyridinyl)-4-phenyl-(2E)-pentenoat,
   Smp.(C-N) 70°C, Klp.(N-I) 75°C;
- 1-(5-Heptyl-2-pyridinyl)-4-phenyl-(2E)-hexenoat,
   Smp.(C-N) 67°C, Klp.(N-I) 87°C;
- 1-(5-Heptyl-2-pyridinyl)-4-phenyl-(2E)-heptenoat,
   Smp.(C-N) 67°C, Klp.(N-I) 79°C;
- 1-(5-Heptyl-2-pyridinyl)-4-phenyl-(2E)-octenoat,
   Smp.(C-N) 58°C, S₂-S (44°C), Klp.(N-I) 87°C;
- 1-(5-Heptyl-2-pyridinyl)-4-phenyl-(2E)-nonenoat;
- 1-(5-Heptyl-2-pyridinyl)-4-phenyl-(2E)-decenoat,
   S₂-N 59°C, Klp.(N-I) 87°C;
- 1-(5-Heptyl-2-pyridinyl)-4-phenyl-(2E)-dodecenoat,
   S₂-S_{C} 57°C, S_{C}-N 66°C, Klp.(N-I) 87°C;
- 1-(5-Octyl-2-pyridinyl)-4-phenyl-(2E)-butenoat,
   Smp.(C-N) 54°C Klp.(N-I) 89°C;
- 1-(5-Octyl-2-pyridinyl)-4-phenyl-(2E)-pentenoat,
   Smp.(C-S₂) 57°C, S₂-N (33°C), Klp.(N-I) 72°C;
- 1-(5-Octyl-2-pyridinyl)-4-phenyl-(2E)-hexenoat;
- 1-(5-Octyl-2-pyridinyl)-4-phenyl-(2E)-heptenoat;
- 1-(5-Octyl-2-pyridinyl)-4-phenyl-(2E)-octenoat,
   Smp.(C-N) 62°C, S_{C}-N(46°C), Klp.(N-I) 83°C;
- 1-(5-Octyl-2-pyridinyl)-4-phenyl-(2E)-nonenoat;
- 1-(5-Octyl-2-pyridinyl)-4-phenyl-(2E)-decenoat;
- 1-(5-Octyl-2-pyridinyl)-4-phenyl-(2E)-undecenoat;
- 1-(5-Octyl-2-pyridinyl)-4-phenyl-(2E)-dodecenoat;
- 1-(5-Nonyl-2-pyridinyl)-4-phenyl-(2E)-butenoat;
- 1-(5-Nonyl-2-pyridinyl)-4-phenyl-(2E)-pentenoat
- 1-(5-Nonyl-2-pyridinyl)-4-phenyl-(2E)-hexenoat;
- 1-(5-Nonyl-2-pyridinyl)-4-phenyl-(2E)-heptenoat;
- 1-(5-Nonyl-2-pyridinyl)-4-phenyl-(2E)-octenoat,
   Smp.(C-N) 62°C, S₂-S_{C} (47°C), S_{C}-N (57°C), Klp.(N-I) 86°C;
- 1-(5-Nonyl-2-pyridinyl)-4-phenyl-(2E)-nonenoat
- 1-(5-Nonyl-2-pyridinyl)-4-phenyl-(2E)-decenoat
- 1-(5-Nonyl-2-pyridinyl)-4-phenyl-(2E)-undecenoat
- l-(5-Nonyl-2-pyridinyl)-4-phenyl-(2E)-dodecenoat

### Beispiel 5

Zur Untersuchung der Eigenschaften der Verbindungen der Formel I wird eine Grundmischung hergestellt und jeweils 20% einer Verbindung der Formel I zugemischt. Die Phasenübergangstemperaturen dieser Mischungen werden bestimmt, die Kristallisationstemperatur T_{C} wird dabei aus Leitfähigkeitsdaten bestimmt. Die Schaltzeiten werden bei 25°C (10 Vpp/µ, Zeit vom Start des Pulses bis zum Maximum des Stromes) gemessen. Die Messwerte sind in der Tabelle l zusammengefasst.

### Grundmischung

- 27,6 Gew.% Bis[(S)-l-methylheptyl]-p-terphenyl 4,4'-dicarbonsäureester
- 18,4 Gew.% 2-[4-(Hexyloxy)phenyl]-5-nonylpyrimidin
- 18,4 Gew.% 2-[4-(Nonyloxy)phenyl]-5-nonylpyrimidin
- 9,2 Gew.% 2-[4-(Nonyloxy)phenyl]-5-octylpyrimidin
- 9,2 Gew.% 2-[4-(Heptyloxy)phenyl]-5-heptylpyrimidin
- 9,2 Gew.% 2-[4-(Decyloxy)phenyl]-5-octylpyrimidin
- 8,0 Gew.% 4-(Decyloxy)benzoesäure-4-(2-[trans-4-pentylcyclohexyl]-l-äthyl)phenylester.

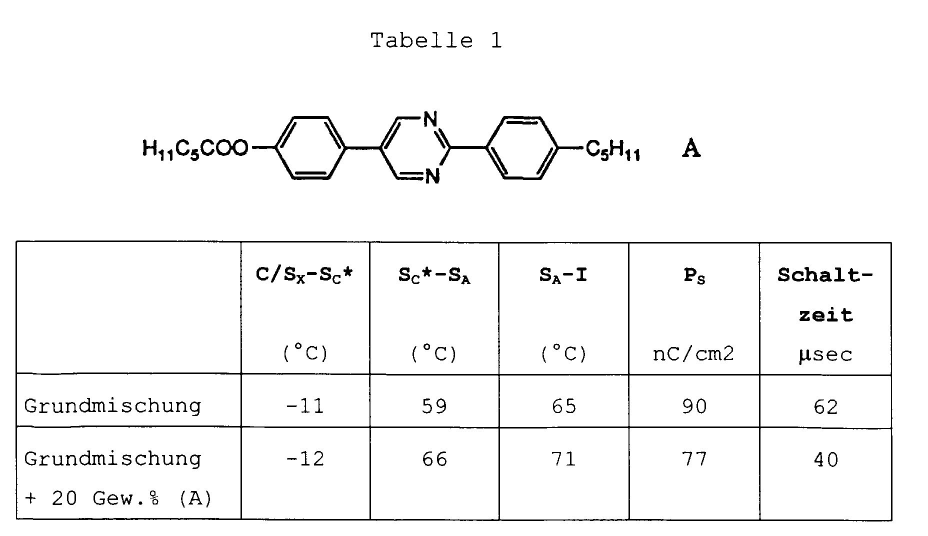

## Patentansprüche

1. Verbindungen der allgemeinen Formel 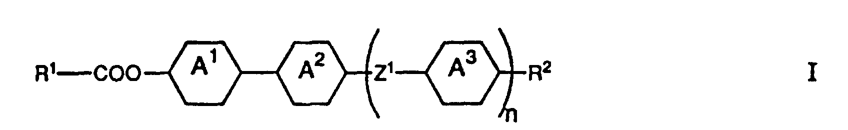 worin
R^{l} unsubstituiertes oder einfach oder mehrfach mit Halogen substituiertes Alkyl oder Alkenyl mit l bis 12, bzw. 2 bis 12 Kohlenstoffatomen, in welchen eine Methylengruppe durch -O- ersetzt sein kann, bedeutet;
R² unsubstituiertes oder einfach oder mehrfach mit Halogen substituiertes Alkyl oder Alkenyl mit l bis 12, bzw. 2 bis 12 Kohlenstoffatomen, in welchen eine oder mehrere Methylengruppen durch -O-und/oder -COO- oder -OOC- ersetzt sein können, bedeutet;
Z¹ eine einfache Kovalenz-Bindung oder -CH₂CH₂-bedeutet;
A¹, A² unabhängig voneinander unsubstituiertes oder einfach oder mehrfach mit Halogen substituiertes 1,4-Phenylen darstellen;
A³ Pyridin-2,5-diyl oder Pyrimidin-2,5-diyl darstellt; und
n 1 ist.

2. Verbindungen gemäss Anspruch l, dadurch gekennzeichnet, dass R² ein Alkyl mit l bis 7 Kohlenstoffatomen oder ein Alkenyl mit 2 bis 7 Kohlenstoffatomen darstellt.

3. Verbindungen gemäss einem der Ansprüche 1 oder 2, dadurch gekennzeichnet, dass R¹ Alkyl oder Alkoxyalkyl bedeutet.

4. Verbindungen gemäss einem der Ansprüche 1 bis 3, mit der allgemeinen Formel 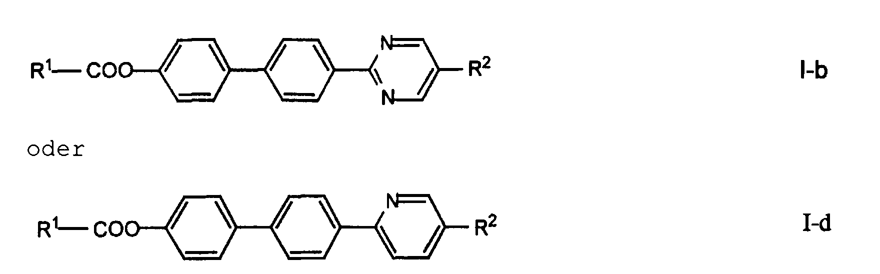 worin R¹ und R² die im Anspruch 1 angegebene Bedeutung haben.

5. Flüssigkristalline Mischung enthaltend mindestens zwei Verbindungen, wovon mindestens eine Verbindung die allgemeine Formel 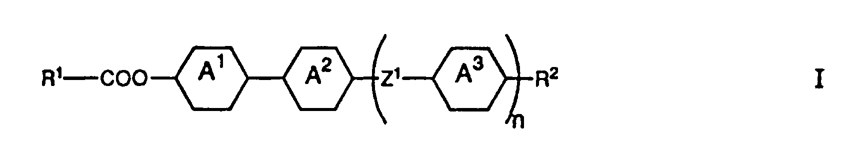 hat, wobei in der Formel I
R^{l} unsubstituiertes oder einfach oder mehrfach mit Halogen substituiertes Alkyl oder Alkenyl mit l bis 12, bzw. 2 bis 12 Kohlenstoffatomen, in welchen eine Methylengruppe durch -O- ersetzt sein kann, bedeutet;
R² unsubstituiertes oder einfach oder mehrfach mit Halogen substituiertes Alkyl oder Alkenyl mit l bis 12, bzw. 2 bis 12 Kohlenstoffatomen, in welchen eine oder mehrere Methylengruppen durch -O-und/oder -COO- oder -OOC- ersetzt sein können, bedeutet;
Z¹ eine einfache Kovalenz-Bindung oder -CH₂CH₂-bedeutet;
A¹, A² unabhängig voneinander unsubstituiertes oder einfach oder mehrfach mit Halogen substituiertes 1,4-Phenylen darstellen;
A³ Pyridin-2,5-diyl oder Pyrimidin-2,5-diyl darstellt; und
n 1 ist.

6. Flüssigkristalline Mischung nach Anspruch 5, dadurch gekennzeichnet, dass es eine ferroelektrische Mischung ist.

7. Verwendung einer Verbindung gemäss einem der Ansprüche 1 bis 4 für elektro-optische Zwecke.

8. Elektrooptische Vorrichtung enthaltend eine Verbindung gemäss einem der Ansprüche 1 bis 4 oder eine flüssigkristalline Mischung gemäss Anspruch 5 oder 6.
